# EUROPEAN PATENT APPLICATION

(11) **EP 4 529 825 A1**
(43) Date of publication of application: **02.04.2025**
(21) Application number: 23199852.7
(22) Date of filing: 26.09.2023
(51) Int. Cl.: A61B 1/00, A61B 1/005, A61B 1/008

(54) **ENDOSCOPE WITH A BENDING SECTION CONTROLLED BY STEERING WIRES**

(71) Applicant: Ambu A/S, 2750 Ballerup (DK)
(72) Inventor: HANSEN, Frederik Clausager Vemb, 2400 Copenhagen NV (DK)
(74) Representative: Winter, Brandl - Partnerschaft mbB

(57) **Abstract**

An endoscope (1) having a bending section (20) comprising segments (22) connected to each other by hinges (24). The bending section has a central passage (12) passing each segment (22) and extends from a distal end to a proximal end, where an inner surface is facing and encircling the central passage (12). At least two grooves (18) into the inner surface extend in parallel with the central passage (12) and in open connection with the central passage. Two steering wires (25) passes in a respective one of the grooves through the bending section. An anchor (15, 15', 15") is provided with a holding part (17, 17') such that displacement of the steering wires (25) from the grooves (18) towards a center of the central passage (12) is blocked.

## Description

### Technical Field

The present disclosure relates to an endoscope comprising an insertion cord including a bending section. Bending of the bending section is controlled by steering wires running in dedicated parts of the bending section.

### Background

Flexible endoscopes for medical purposes are often provided with a bending section at a distal end of an insertion tube. This enables the user of the endoscope to maneuver the distal tip of the endoscope inside the human anatomy, such as in the airways, in the kidneys or the gastro-intestinal system, and e.g., to study or perform procedures at tissue of interest. The bending section is typically bent by pulling steering wires. If the endoscope is a 2-way bending endoscope, i.e., bending in two opposite directions but in the same plane, the bending section will typically be controlled by two steering wires, which are controlled by one steering wire actuator arranged in the handle of the endoscope, allowing the user to bend the bending section by adjusting a bending lever. If the endoscope is a four-way bending endoscope, i.e., also bending in two opposite directions in a second plane perpendicular to the first mentioned plane, the bending section will typically be controlled by four steering wires, where one steering wire actuator controls two steering wires for bending in the first plane, and the other steering wire actuator controls the two other steering wires for bending in the second plane. Typically, each steering wire actuator is controlled by a rotational wheel on the endoscope handle.

The steering wires will pass from the handle to the proximal end of the bending section inside wire pipes, which are tubes having limited compressibility. Thereby so-called Bowden cables are formed. The wire pipes are at their proximal end connected inside the handle. At the distal end of the wire pipes, they are connected to the proximal end of the bending section, whereas the steering wires continue through the bending section, often in dedicated lumens. The wire pipes are held stationary and translatory movement of the steering wire at the proximal end is transmitted to the distal end as a corresponding translatory movement of the steering wire relative to the wire pipe.

For single use endoscopes the bending section may be molded in one piece from a polymer material. Especially for narrow bending sections for endoscopes with a small outer diameter, this molding process can be difficult. E.g., for ureteroscopes, often having a relatively long bending section and an outer diameter of maybe less than 3 mm, it may be difficult to manufacture, and find space for, closed lumens for the steering wires. Especially the long and thin cores needed for the steering wire lumens in the molding process, will be fragile and could cause frequent interruptions of the molding process.

It is simpler to manufacture a bending section with only one inner lumen having grooves or recesses functioning as channels for the steering wires. Such grooves are in open connection with the rest of the lumen. An example of this can be found in EP 3 925 513 A1. However, this design of the bending section does involve a minor risk that the steering wire may be displaced from the dedicated channel during a procedure involving specific maneuvering, e.g., inside the kidney. Such maneuvering could be that the proximal end of the bending section is bent in one direction and the operator of the endoscope manipulates the steering wire actuator to bend the distal end of the bending section in the opposite direction.

### Summary

It is an object of the present disclosure to solve the above problem by providing an endoscope comprising a bending section having an inner lumen extending in a longitudinal direction. Grooves are extending from this inner lumen and steering wires are arranged in these grooves. An anchor is placed such that displacement of the steering wire out of the groove is prevented.

The endoscope may have a bending section comprising segments connected to each other by hinges. The bending section has a central passage passing each segment and extends from a distal end to a proximal end, where an inner surface is facing and encircling the central passage. At least two grooves into the inner surface extend in parallel with the central passage and in open connection with the central passage. Two steering wires passes in a respective one of the grooves through the bending section. An anchor is provided with a holding part such that displacement of the steering wires from the grooves towards a center of the central passage is blocked.

The endoscope may comprise a handle and an insertion cord extending from the handle and including a bending section molded in one piece, e.g., from a fused polymer, the bending section comprises:
- Segments connected to each other by hinges, the hinges allow two neighboring segments to bend relative to each other in a bending plane, the segments and the hinges forms an outer surface of the bending section.
- A central passage passing each segment and extends from a distal end to a proximal end of the bending section, where each segment has a circumferential wall, the circumferential wall has an inner surface facing and encircling the central passage.
- At least two grooves extend from the inner surface into the circumferential wall, the grooves extending in parallel with the central passage and in open connection with the central passage, the central passage and the grooves extending through the segments of the bending section. The grooves may be cut-outs in the inner surface of the circumferential wall of the segments but may not extend all the way in the longitudinal direction of the bending section, as there may not be groove or cut-out in the parts where hinges connect two neighboring segments.
- An indent extends from the outer surface into at least one segment or in at least one hinge.

The endoscope further comprising two steering wires extending from the handle to the distal end of the bending section, each steering wire passes in in a respective one of the grooves through the bending section. Further, an anchor is arranged in the indent, the anchor being provided with a holding part such that displacement of the steering wires from the grooves towards a center of the central passage is blocked.

One advantage of the one or more anchors is that it enables a small outer diameter bending section for an endoscope molded in one piece and with one inner lumen, where the risk of having the steering wires displaced during an endoscopic procedure is eliminated, or at least reduced.

In a variation of the present embodiment, the anchors are placed in at least two indents in positions in segments, or next to segments, placed in only in the first 60 % of the bending section, i.e., the 60% of the bending section length starting from the proximal end of the bending section and extending towards the distal end of the bending section. It has been found that placing the anchors in this first proximal part of the bending section is advantageous, since the force on the steering wires in the direction towards a center of the central passage of the bending section, has found to be largest in this first proximal part. In general, it is also preferred to keep the total number of anchors low to keep the manufacturing costs as low as possible. Preferably, anchors should be applied in, or in connection with less than half of the segments. Preferably, anchors should be applied in, or in connection with less than a third of the segments. In a further variation anchors are arranged in at least two segments, and in less than 40% of the segments, alternatively in less than 30% of the segments.

In a variation of the present embodiment, at least two segments are provided with anchors such that displacement of the two steering wires is blocked. Having the anchors within the segments, provides a very stable positioning of the anchors.

In a variation of the present embodiment, at least two segments provided with anchors are separated by at least one segment without an anchor. It has been found to provide a good trade-off between manufacturing costs and stability of the bending section with respect to the steering wires not being displaced, to have the anchors spread out, and not in neighboring segments. In a further variation there may be at least two segments provided with anchors which are separated by at least two segments without an anchor.

In a variation of the present embodiment, each anchor has a holding part extending into a space where the groove and the central passage meet. The holding part has the advantage that it provides a direct blockage preventing displacement of the steering wire.

In a variation of the present embodiment, the steering wires in the bending section are passing inside coil springs, the coil springs with the steering wires are each arranged in a groove, and the anchor is arranged to block displacement of the coil springs, and thereby the steering wires, from the grooves towards a center of the central passage. This reduces the risk of the steering wires moving into the narrow space between the working channel and the inner surface of the bending section, especially in parts of the bending section where there are no anchors.

In a variation of the present embodiment, the anchors have an exterior surface being level with an outer surface of a segment and the anchors have an interior surface being level with the inner surface. This enables that the anchor may have sufficient dimensions to provide sufficient mechanical strength to resist the forces from the steering wire, e.g., via the coiled spring.

In a variation of the present embodiment, the anchors are made from hardened glue. This enables a simple and cost-effective manufacturing of the endoscope and has also been found to result in a very stable and efficient anchor. In a further variation each anchor is adhering to a segment of the bending section.

In a variation of the present embodiment a ledge is extending from the segment between the indent and the central passage, the ledge is supporting the anchor. This provides a strong and stable support for especially the glued anchor variation.

In a variation of the present embodiment the anchors are made from a metal disc. This provides an anchor which takes up a minimum amount of space in the proximal to distal length direction of the bending section.

In a second aspect the disclosure relates to a method for assembling an endoscope, comprising:
- providing a bending section molded in one piece from a fused polymer, and having:
   ∘ segments connected to each other by hinge members, the hinges allowing two neighboring segments to bend relative to each other in a bending plane, the segments and the hinges forms an outer surface of the bending section,
   ∘ a central passage passing each segment and extending from a distal end to a proximal end of the bending section, where each segment has a circumferential wall, the circumferential wall has an inner surface facing and encircling the central passage,
   ∘ at least two grooves extending from the inner surface into the circumferential wall, the grooves extending in parallel with the central passage and in open connection with the central passage, the central passage and the grooves extending through the segments of the bending section,
   ∘ an indent extending from the outer surface into at least one segment or in at least one hinge;
- arranging two steering wires to extend from the handle to the distal end of the bending section, such that each steering wire passes in a separate groove through the bending section;
- arranging an anchor in the indent, where the anchor is provided with a holding part such that displacement of the steering wires from the grooves towards a center of the central passage is blocked.

This method has been found to be a cost-effective way to manufacture bending sections for endoscopes, e.g., single use endoscopes, having a small diameter insertion cord. The outer diameter of the insertion cord, including the bending section, may be 3 mm or smaller.

In a third aspect the disclosure relates to a system comprising an endoscope according to the first aspect and variations thereof, a monitor and a control unit.

Segments of the bending section are including a proximal end segment, a distal end segment and intermediate segments arranged between the proximal end segment and the distal end segment. The segments are not necessarily identical. Especially, the proximal end segment and the distal end segment may have features enabling the connection of the bending section to the insertion tube and to the distal tip housing, respectively.

In this disclosure, the expression "distal" is defined to be in the direction toward the patient, and "proximal" is defined to be in the direction away from the patient. For the handle of the endoscope, the distal end will be the end where the insertion tube is connected, and the proximal end is the opposite end. Further, the expression "handle" may be a positioning interface, or interface, which functions to control the position of the insertion cord and operating the bending section. The handle, or positioning interface, may be an interface operated by a robotic arm, or it may be a handle operated by the hand of an endoscope user.

For this disclosure one steering wire is counted as one passage from the steering wire actuator (or roller) to the distal end of the bending section. I.e., if the same unbroken steering wire continues from the steering wire actuator to the distal end of the bending section and back to the steering wire actuator, and one part is applied for bending for example to one side and the other part is applied for bending to the opposite side, this is counted as two steering wires, e.g., first and second steering wires.

An anchor arranged in connection with a segment may be able to keep both steering wires inside the grooves, or an anchor in a segment may only keep one steering wire in the corresponding groove. In the later situation two anchors in the same segment may be preferred, but anchors for the two different steering wires could also be placed in different segments.

### Brief description of the figures

The above-mentioned embodiments and variations, features and advantages thereof will be further elucidated by the following illustrative and nonlimiting detailed description of embodiments disclosed herein with reference to the appended drawings, wherein:
Fig. 1 shows an endoscope and a monitor with a control unit.
Fig. 2 shows an example of a steering wire actuator, here in the form of a roller, and a wire pipe fastener.
Fig. 3 shows a distal end of an endoscope including a bending section.
Fig. 4 shows a cross-sectional view of a bending section provided with a glued anchor.
Fig. 5 shows a part of a bending section where one segment is provided with anchor.
Fig. 6 shows the bending section of fig. 5 from a different angle.
Fig. 7 shows a part of a bending section with a disc anchor.
Fig. 8 shows an example of a separate disc anchor.
Fig. 9 shows a cut through bending section with the disc anchor from fig. 8.
Fig. 10 shows a part of a bending section with a different disc anchor.
Fig. 11 shows a part of a bending section with part of segments removed to give a better view of the disc anchor.
Fig. 12 shows a cross-sectional view of a bending section provided with the disc anchor of Fig. 10.
Fig. 13 shows the disc anchor included in fig. 10 - 12.
Fig. 14 schematically illustrates S-shaped curvature of a bending section.

In the drawings, corresponding reference characters indicate corresponding parts, functions, and features throughout the several views. The drawings are not necessarily to scale and certain features may be exaggerated in order to better illustrate and explain the disclosed embodiments. For simplicity, this disclosure will focus on a two-way bending endoscope, but the disclosure is relevant for, and covers, also a four-way bending endoscope.

### Detailed description

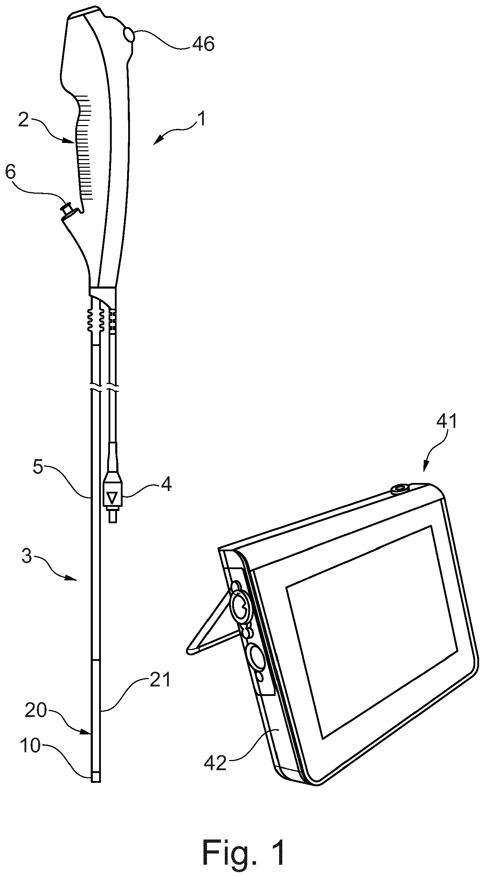

Fig. 1 illustrates an endoscope 1, which comprises a handle 2, an insertion cord 3 and an electrical cable with a connector 4 for connecting the endoscope 1 to a monitor 41. The insertion cord 3 is the part to be inserted into a body lumen during an endoscopic procedure. The insertion cord comprises a distal tip 10, a bending section 20 and a main tube 5. The handle 2 may comprise an entrance to a working channel 6 running through the insertion cord to the distal tip. The handle also comprises a bending lever 46, which can be used for bending the bending section.

The distal tip 10 comprises a camera and light emitters, e.g., in the form of one or more LEDs or the end of an optical light fiber.

The monitor 41 may be combined with an electronic circuit for receiving and processing the image stream from the camera as well as a processor for image processing, user interface, storage of images etc. But the monitor part and the electronic circuit and processor part may also be separate parts. The electronic circuit and the processor part are also referred to as a control unit 42.

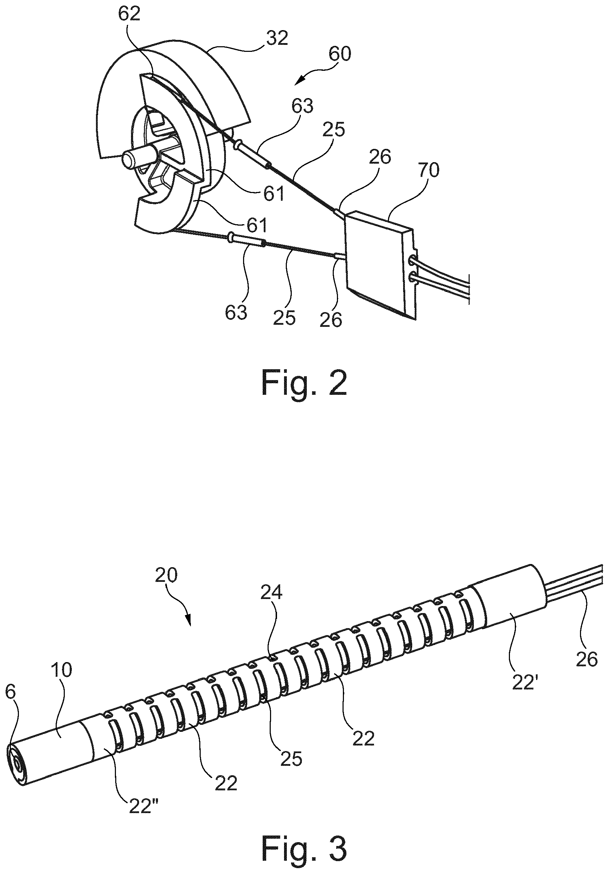

Fig. 2 shows a schematic example of a roller 32 for a steering wire actuator 60 configured for bending the bending section by pulling the steering wires 25. The steering wires 25 are moved by rotation of wire drum curved surfaces 61, which are a circular arc surfaces or a curved surface placed on the roller 32 and supporting the steering wires. In the example, one end of the steering wire has been drawn through a fixing structure 62 and bent back and fastened to itself by a crimp 63. The proximal ends of the wire pipes 26 are fixated in the wire pipe fastener 70, whereas the distal ends of the wire pipes are secured to the proximal end of the bending section 20. Both the wire pipe fastener 70 and the roller 32 are often secured in the handle 2. The system in fig. 2 is for a two-way bending endoscope.

Fig. 3 shows a distal end of the insertion cord 3 of an endoscope, where the bending cover (not shown) typically covering the bending section 20 part and often makes this part watertight, has been removed. The proximal end of the bending section is attached to the main tube 5, and the distal end of the bending section is attached to the distal tip 10. In this example the bending section is molded in one piece and comprises a number of segments 22. A proximal end segment 22' is connected to the distal end of the main tube 5. A distal end segment 22" is connected to the distal tip 10. The segments are held together by hinges 24, so that the segments can be bent relative to each other by manipulation of the steering wires 25. An example of such a bending section molded in one piece can be seen in EP 3 925 513 A1, which is incorporated herein by reference in its entirety.

Alternatively, the bending section 20 could be extruded in a relatively soft and resilient material, e.g., a foam-like material, with lumens for steering wires, electrical wires and the tubes passing through.

Steering wires 25 are connected in a fixed connection to the distal end, e.g., the distal end bending segment 21. I.e., the steering wire is preferably not movable in relation to the distal end bending segment. Between the handle and the distal end of the main tube 5, or the proximal end of the bending section 20, the steering wire 25 is guided in a wire pipe 26 which is secured in the handle 2 distal to the steering wire actuator 60. Other designs of the bending section are also possible.

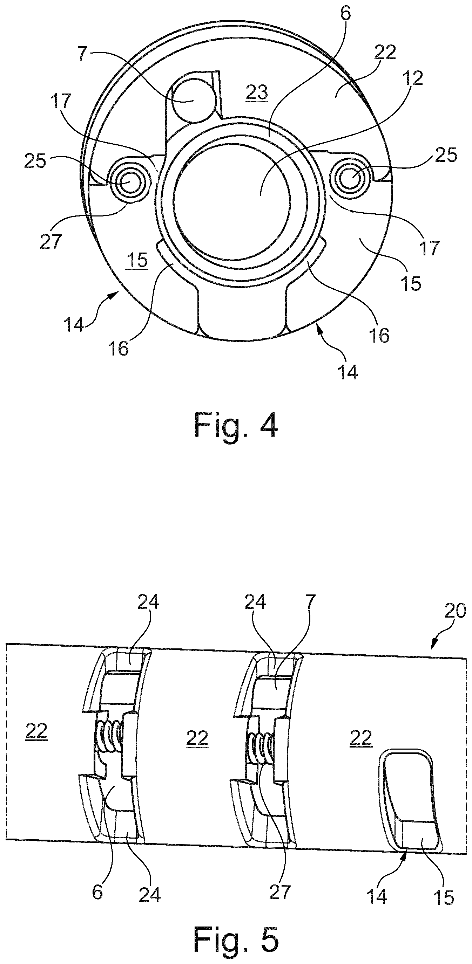

Fig. 4 - 6 shows an embodiment of a bending section 20 for an endoscope 1, where the bending section 20 is provided with only one inner lumen or central passage 12. This central passage 12 has grooves 18 or recesses in open connection with the rest of the central passage 12. The steering wires 25 are arranged in these grooves 18. An anchor 15 is provided to avoid that the steering wire 25 is displaced from, or leaves, the groove 18, during bending operations. The cross-sectional view in fig. 4 is made at the position of the glued anchor 15, and the indent 14, in fig. 5.

The anchor 15 shown in fig. 4-6 may be made from a hardened adhesive which fills up an indent 14 made in a segment 22. Fig. 4 shows a cross-sectional view of a segment 22, where two indents 14 are made. The indents have been filled with a glue or adhesive, which have been hardened, and thereby forming the anchors 15. Fig. 5 shows the bending section 20 from one side, whereby one indent 14 in a segment 22 can be seen. An anchor 15 made from a hardened transparent glue is placed in this indent 14. Fig. 4 shows that a similar anchor 15 may be placed at two opposite sides of the bending section 20. One anchor for each steering wire 25.

The anchors 15 have a holding part 17 extending into the space between the steering wire 25 and the working channel 6. These holding parts 17 of the anchors are preventing displacement of the steering wires 25 in the direction toward the working channel 6.

It is shown in fig. 4 - 6 that the steering wires in the bending section may be arranged inside coil springs 27. The coil springs 27 with the steering wires 25 are each arranged in a groove 18, and the anchor 15 is arranged to block displacement of the coil springs 27, and thereby the steering wires 25, from the grooves 18 towards a center of the central passage 12. This reduces the risk of the steering wires moving into the narrow space between the working channel 6 and the inner surface of the bending section 20. This may be advantageous for the segments 22 where no anchors 15 are arranged. If the springs 27 are part of the design, the holding part 17 of the anchors will extend into the space between the springs 27 and the working channel 6.

The use of springs 27 for guiding or enclosing the steering wires through a bending section where grooves 18, i.e., the dedicated lumen for steering wires, are in open connection to the lumen for the working channel, is described further in EP 4 035 584 A1, which is incorporated herein by reference in its entirety, and works for a two-way bending endoscope.

The spring 27 is made from a wound wire, e.g., a steel wire. The distance between two neighboring windings of the spring 27 may be referred to as the pitch of the spring 27. So, the minimum pitch is similar to the diameter of the wire used for the spring. The pitch should be selected so that the springs can both be compressed and extended during bending of the bending section. The pitch may also vary, so that it is low in segments where an anchor 15 is placed, and higher in other positions. A low pitch may prevent liquid glue from entering between the windings and adhere to the steering wire during the gluing process providing a glued anchor. The pitch may be at minimum in places where an anchor is to be placed. However, if the liquid glue has a high viscosity, the pitch may be larger than minimum and still prevent the glue from getting in contact with the steering wire.

The steering wires 25 may, alternatively, be moveably arranged through the anchors 15 without the springs 27. In that case, it must be ensured that there is no adherence between the steering wire and the anchors in manufacturing. This can be ensured by applying a lubricant to the steering wire 25. This may be applied before application and hardening of the glue. The glue and the steering wire material may also be selected so that there will be no adherence. In that case the lubricant might not be necessary but may still be advantageous for lowering the friction between steering wire and hardened glue.

The anchors 15 shown in fig. 4 - 6 are relatively large compared to the size of the holding part 17 actively holding the steering wire in the grooves 18. One reason for this is that bending sections 20 molded in one piece from a polymer material are often made from POM, and most glues does not adhere very well to POM. Therefore, the geometry of the anchor 15 made from hardened glue, may be such that the anchor is kept in the correct position in the indent 14 of the segment 22, also if the glue does not adhere to the bending section material. This may be achieved by a ledge 16 extending between part of the anchor 15 and the working channel 6. The ledge 16 may be formed in one piece with the segment and the rest of the bending section and may be molded from the same fused polymer material. The ledge 16 can thus support the anchor 15 and is adapted to keep the anchor 15 in the correct position.

Also, to the extent that there is some adherence between the bending section material and the glue anchor 15, increasing the contact area between the two, which is also done by the ledge 16, will improve the stability of the anchor position.

For the glued anchor the indent may be placed in a segment with the segment limiting the indent both in the distal and in the proximal direction. This may make it simpler to control where the glue is flowing during the gluing process.

It should be noted that the bending section 20 may often be provided with a bending cover 21 on the external side. This provides a watertight barrier towards the surroundings of the endoscope insertion cord 3. This bending cover 21 may have some effect on supporting the anchor 15 in the correct position. The effect may, however, be relatively small as the bending cover is often very flexible and not glued to the bending cover.

For the glue anchor a UV curing glue may be applied, as curing can happen immediately, and thereby limiting the time for the glue to flow into unwanted places inside the bending section. The glue for the anchor 15 may be applied from the outside of the bending section 20, e.g., using a small needle tip for precise application. Viscosity of the glue may be selected to be low enough for the glue to flow in between the steering wire 25 and the working channel 6 to form the holding part 17. Also, the viscosity of the glue may be selected to be high enough to avoid glue flowing into unwanted parts of the bending section via capillary forces. Also, it may be easier to create a smooth surface on the outside of the anchor 15, i.e., the external side of the bending section, with a higher viscosity glue with higher surface tension.

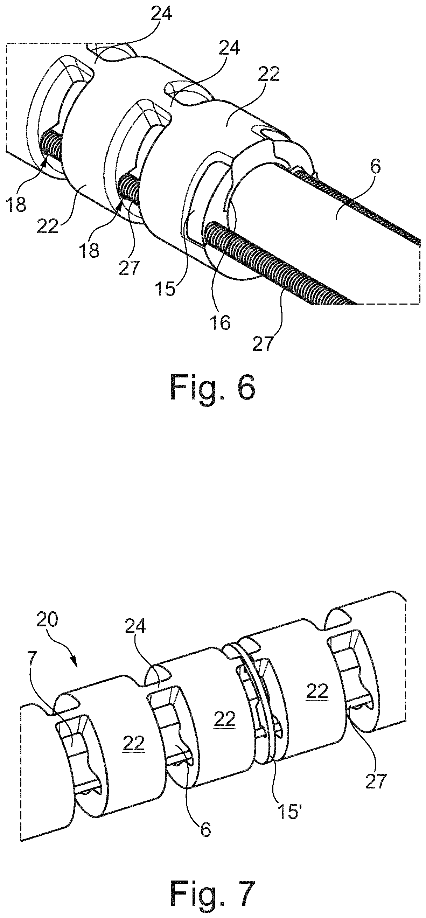

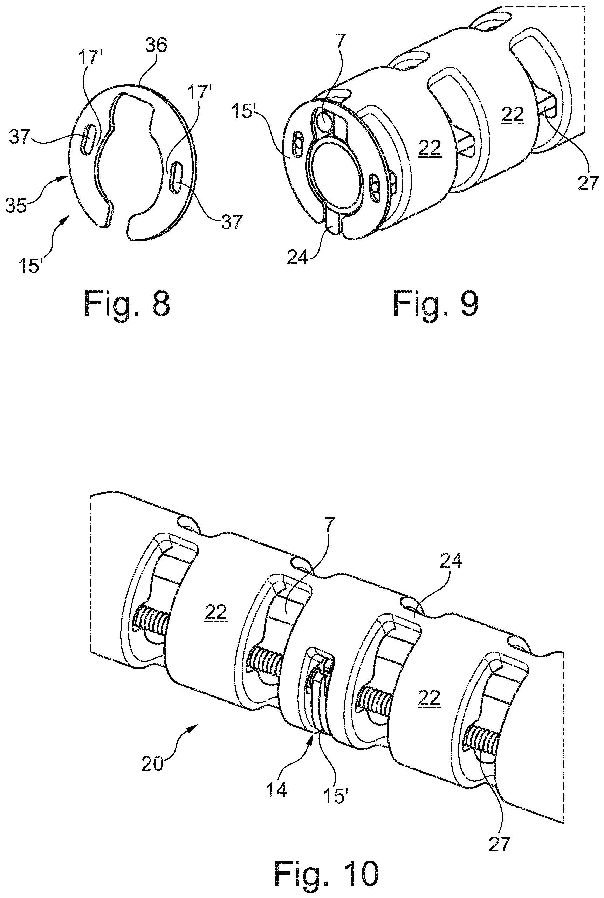

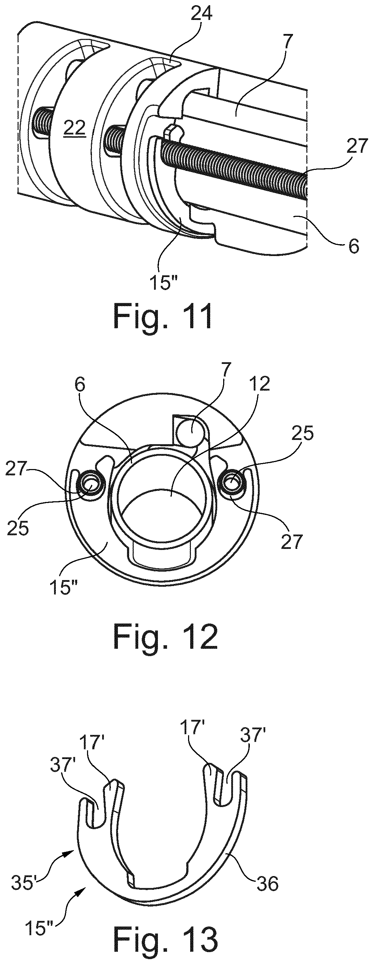

Fig. 7-12 shows an embodiment where the anchor 15' is made from a disc 35. The shape of the disc may be like the Greek letter omega, of horse-shoe shaped, or U-shaped. This disc is arranged in an indent 14, where the indent may be in a segment 22 as shown in fig. 10 and 11, or the indent may be in a hinge 24 connecting two segments 22, as shown in fig. 7 and 9. Preferably, the disc 35 may comprise an anchor part 15' for both of the two steering wires, or, alternatively, the disc 35 may only have an anchor part for one of the steering wires 25.

The disc 35 is preferably made from a hard material, e.g., a metal such as steel. The disc may also be made from a relatively hard polymer, e.g., PC, PS, PA. The thickness of the disc may depend on the material. For steel the thickness may be in the range 0.1 - 0.5 mm, preferably 0.1 - 0.3 mm. If the material is a polymer the thickness may be above 0.3 mm.

The disc 35 may be shaped so that an outer circumference 36 of the disc is level with an outer surface formed by the parts of the bending section segments 22 and the hinges 24 facing opposite to a central passage 12 of the bending section 20. This outer surface may also in this embodiment be covered by a bending cover 21.

The disc 35 is provided with a holding part 17' extending into the space between the steering wire 25 and the working channel 6 such that displacement of the steering wire 25, and maybe also the spring 27, in a direction towards the working channel 6, or a center of the central passage is prevented.

When the disc 35 is arranged in an indent 14 in a hinge 24, this may limit the angle by which the two neighboring segments, being next to this hinge, may be able to bend relative to each other. For some applications of the endoscope such a limitation may be acceptable. If the bending section is provided with many hinges, e.g., 20 or more, this limited bendability of maybe two pair of hinges, may not affect the overall bending performance significantly.

Fig. 8 and 9 shows a variation of the embodiment where the anchor 15' is a disc 35. In this variation passages 37 for the steering wires, and e.g., also for the springs, are provided as closed holes. This means that the steering wires and e.g., the springs will need to be threaded through these holes.

Alternatively, another variation of the anchor 15" provided as a disc 35, is shown in fig. 10 - 13. In this variation the passages 37' are not closed, but open at one side, so that the disc 35 can be inserted into the indent 14 after the steering wires 25, and e.g., the springs 27, have been arranged in the grooves for the steering wires 25, and e.g., the springs 27. In this variation the indents are preferably made in a segment 22 of the bending section.

It is a possible to combine different types of anchors at different positions in the same bending section. The anchors could in general both be the examples provided above, but it could also be other types or designs of anchors.

The number and positions of anchors of anchors along a proximal-distal direction of the bending section, may depend on the total length of the bending section, the outer diameter of the bending section, the maximum bending angle of the distal end of the bending section relative to the proximal end of the bending section, and on the necessary pulling force on the steering wires to obtain this bending angle.

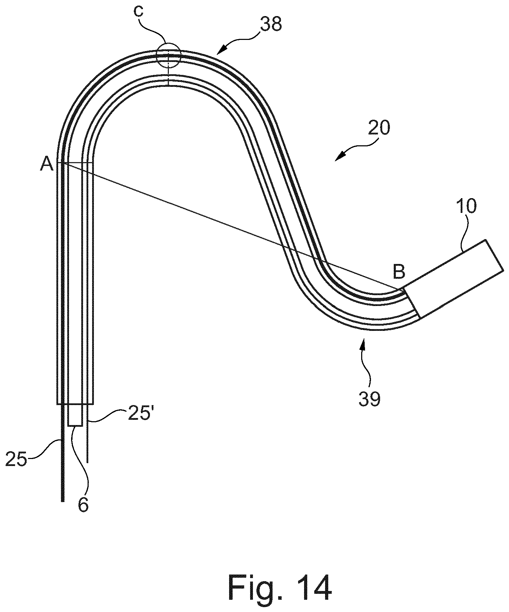

For example, an ureteroscope may have a bending section with a length of more than 35 mm, maybe more than 45 mm. The outer diameter may be around 3 mm or below 3 mm. The maximum bending angle may be around 270 degrees, maybe in the range 225 - 270 degrees. When a ureteroscope is used in a procedure in a kidney, it may often happen that the bending section has a first curvature 38 in the proximal half length of the bending section going in one direction, and at the same time needs to bend the distal end of the bending section in the opposite direction, resulting in a second curvature 39. This is illustrated schematically in fig. 14, where a resulting S-shaped curvature of the bending section can be seen.

This means that there will be a relatively high force on the steering wire 25 to the convex side of the first curvature 38 or proximal bending curve, as this steering wire 25 needs to be pulled to bend the distal end of the bending section into the second curvature 39. This force is directed towards a center of the central passage of the bending section. In general, the force on the steering wire 25 will tend to move it towards the line A-B. The circle c indicates the area where the force towards the line A-B is maximum for the steering wire 25. Therefore, anchors 15, 15',15" may be placed in the part of the bending section where this area with the maximum force is likely to be during a procedure.

Looking at the bending of the bending section shown in fig. 14, it is realized that the other steering wire 25' placed to the concave side of the first curvature 38, will be affected by a relatively smaller force, since this steering wire does not have to be pulled to bend the distal end of the bending section into the second curvature 39. Also, the force on the other steering wire 25' in the first curvature 38 is directed towards the groove 18 and the circumferential wall 23 of the segments 22, which will be more resistant towards any unintended displacement of the steering wire compared to the working channel 6.

The above-mentioned embodiments or variations of the anchor, and how the anchor is arranged and connected to the bending section, are not limiting for the scope of this disclosure. Other designs, arrangements and connections of the anchor are possible within the scope of the claims.

### List of references

| | | | |
|---|---|---|---|
| 1 | endoscope | 23 | circumferential wall |
| 2 | handle | 24 | hinge |
| 3 | insertion cord | 25, 25' | steering wire |
| 4 | electrical cable with plug | 26 | wire pipe |
| 5 | main tube | 27 | spring |
| 6 | working channel | 32 | roller |
| 7 | electrical wire bundle | 35, 35' | disc |
| 10 | distal tip | 36 | outer circumference |
| 12 | central passage | 37, 37' | passages |
| 14 | indent | 38 | first curvature |
| 15, 15', 15'' | anchor | 39 | second curvature |
| 16 | ledge for supporting anchor | 41 | monitor |
| 17, 17' | holding part | 42 | control unit |
| 18 | groove | 46 | bending lever |
| 20 | bending section | 60 | steering wire actuator |
| 21 | bending cover | 61 | wire drum curved surface |
| 22 | segment | 62 | fixing structure |
| 22' | proximal end segment | 63 | crimp |
| 22" | distal end segment | 70 | wire pipe fastener |

## Claims

1. An endoscope comprising:
- a handle;
- an insertion cord extending from the handle and including a bending section molded in one piece, the bending section comprising:
i. segments connected to each other by hinges, the hinges allowing two neighboring segments to bend relative to each other in a bending plane, the segments and the hinges forms an outer surface of the bending section,
ii. a central passage passing each segment and extending from a distal end to a proximal end of the bending section, where each segment has a circumferential wall, the circumferential wall has an inner surface facing and encircling the central passage,
iii. at least two grooves extending from the inner surface into the circumferential wall, the grooves extending in parallel with the central passage and in open connection with the central passage, the central passage and the grooves extending through the segments of the bending section,
iv. an indent extending from the outer surface into at least one segment or in at least one hinge;
- two steering wires extending from the handle to the distal end of the bending section, each steering wire passes in a respective one of the grooves through the bending section;
wherein an anchor is arranged in the indent, the anchor being provided with a holding part such that displacement of the steering wires from the grooves towards a center of the central passage is blocked.

2. The endoscope according to claim 1, wherein anchors are placed in at least two indents in segments, or next to segments, placed in the 60% of the bending section length extending from the proximal end of the bending section towards the distal end.

3. The endoscope according to claim 2, wherein at least two segments are provided with anchors such that displacement of the two steering wires is blocked.

4. The endoscope according to claim 2 or 3, wherein at least two segments provided with anchors are separated by at least one segment without an anchor.

5. The endoscope according to claim 1, wherein each anchor has a holding part extending into a space where the groove and the central passage meet.

6. The endoscope according to claim 1, wherein the steering wires in the bending section are passing inside coil springs, the coil springs with the steering wires are each arranged in a groove, and the anchor is arranged to block displacement of the coil springs, and thereby the steering wires, from the grooves towards a center of the central passage.

7. The endoscope according to claim 1, wherein the anchors having an exterior surface being level with an outer surface of a segment and the anchors having an interior surface being level with the inner surface.

8. The endoscope according to claim 1, wherein the anchors are made from hardened glue.

9. The endoscope according to claim 8, wherein a ledge is extending from the segment between the indent and the central passage, the ledge is supporting the anchor.

10. The endoscope according to claim 1, wherein the anchors are made from a metal disc.

11. The endoscope according to claim 1, wherein anchors are arranged in at least two segments, and in less than 40% of the segments, alternatively in less than 30% of the segments.

12. A method for assembling an endoscope according to any one of the previous claims, comprising:
- providing a bending section molded in one piece from a fused polymer, and having:
i. segments connected to each other by hinge members, the hinges allowing two neighboring segments to bend relative to each other in a bending plane, the segments and the hinges forms an outer surface of the bending section,
ii. a central passage passing each segment and extending from a distal end to a proximal end of the bending section, where each segment has a circumferential wall, the circumferential wall has an inner surface facing and encircling the central passage,
iii. at least two grooves extending from the inner surface into the circumferential wall, the grooves extending in parallel with the central passage and in open connection with the central passage, the central passage and the grooves extending through the segments of the bending section,
iv. an indent extending from the outer surface into at least one segment or in at least one hinge;
- arranging two steering wires to extend from the handle to the distal end of the bending section, such that each steering wire passes in a respective one of the grooves through the bending section;
- arranging an anchor in the indent, where the anchor is provided with a holding part such that displacement of the steering wires from the grooves towards a center of the central passage is blocked.

13. A system comprising an endoscope according to any one of claims 1-11, a monitor and a control unit.
